# EUROPEAN PATENT APPLICATION

(11) **EP 0 638 583 A1**
(43) Date of publication of application: **15.02.1995**
(21) Application number: 94112381.2
(22) Date of filing: 08.08.1994
(51) Int. Cl.: C07H 3/02, C07H 5/04, C07H 5/06, A61K 31/70, A61K 47/48, C07K 5/08, C07H 23/00

(54) **Aminosugar active substances, a process for their preparation and use as pharmaceuticals**

(30) Priority: 13.08.1993 EP 93710014; 16.12.1993 DE 4343001
(71) Applicant: HOECHST AKTIENGESELLSCHAFT, D-65929 Frankfurt am Main (DE)
(72) Inventor: Vertesy, Lászlo, Dr., D-65817 Eppstein (DE); Aretz, Werner, Dr., D-61462 Königstein (DE); Fehlhaber, Hans-Wolfram, Dr., D-65510 Idstein (DE)

(57) **Abstract**

Compounds of the formula I,

X-B-Y (I)

where X is a carrier residue, B is a linker, which may also be omitted, and Y is an aminomonosaccharide residue, excepting salmycins A, B, C and D, have an antibacterial effect.

## Description

The invention relates to compounds composed of an aminosugar, a linker and a carrier molecule, to their preparation and use as pharmaceuticals.

EP Application No. 93118511.0 describes antibiotics, called salmycins, which are composed of an iron chelate (siderophore), a monosaccharide and an aminomonosaccharide. The structure of the salmycins is represented in the following formula.

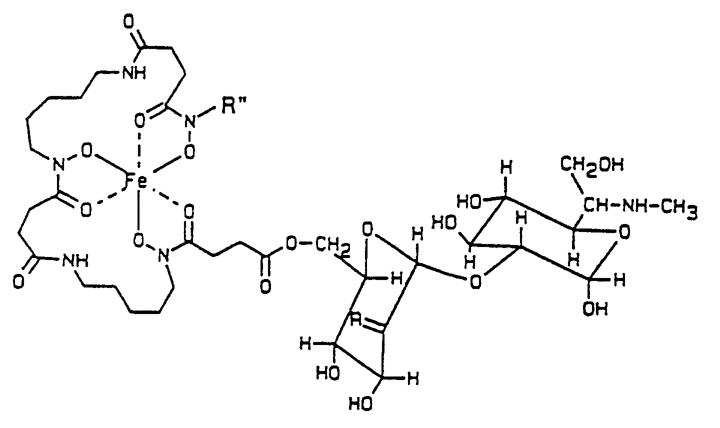

| | | |
|---|---|---|
| Salmycin A: | R = NOH, | R'' = -(CH₂)₅-OH, |
| Salmycin B: | R = O, | R'' = -(CH₂)₅-OH, |
| Salmycin C: | R = O, | R'' = -(CH₂)₄-OH, |
| Salmycin D: | R = NOH, | R'' = -(CH₂)₄-OH. |

Found as constituent of iron-containing antibiotics are hydroxamatoiron(III) complexes or chelates of catechol derivatives. These iron complexes are called siderophores. The siderophores are reviewed by B.F. Matzanke et al. in "Iron carriers and iron proteins", Th.M. Loehr (Ed.), VCH Publishers, 1989, pages 1-121. Siderophores are used by bacteria to transport iron into the cell. The cell membrane has various specific types of gates for active transport of substances. Examples of bacterial transport systems are to be found in: H. Zähner, Angew. Chem. 89 (1977) 696-703. Some of these transport systems are a component of naturally occurring antibiotics, with whose aid the antibiotics get into the bacterial cell. On the basis of these findings there have been attempts to obtain artificial antibiotics by combining a transport system with an active substance. Thus, H. Zähner et al. (The Japanese Journal of Antibiotics, Vol. XXX Suppl. (1977) S-201) describe the chemical coupling of a ferrioxaminyl B or a ferricrocinyl radical, both of which are siderophores, to sulfonamides. A. Brochu et al. (Antimicrobial Agents and Chemotherapy 36 (1992) 2166-2175) combine certain siderophores with cephalosporins. The linkage of a carrier system to an active substance frequently leads to compounds without antibiotic activity.

It has now been found, surprisingly, that replacement of the iron chelate component in salmycins A-D by another siderophore leads to compounds which have antibiotic effects.

Besides the replacement of the iron chelate component in the salmycins it is also possible to replace the mono-saccharide residue, which is the bridge between the siderophore and the aminosugar unit, by another compound. The substances which are formed are accordingly composed of an iron chelate component, of a bridging compound (linker) and of the aminosugar unit.

The invention consequently relates to a compound of the formula I

X-B-Y (I)

in which X is a carrier residue, B is a linker, which may also be omitted, and Y is an amino-monosaccharide residue, excepting salmycins A, B, C and D.

A carrier residue is intended to mean any suitable, coupled transport system. The carrier residue is preferably a siderophore such as, for example,
- the ferrichromes, which include ferricrocin, ferrichrysin, ferrirhodin, ferrichrome A and ferrirubin,
- the ferrioxamines, which include ferrioxamine B and ferrioxamine G,
- N⁵-acetyl-N⁵-hydroxy-L-ornithine tripeptide siderophores and siderophores occurring in albomycins,
- phenols or catechol-containing derivatives such as, for example, spermidine-catechol siderophores (see A. Brochu et al., Antimicrobial Agents and Chemotherapy 36 (1992) 2166-2175).

Particularly preferred carrier residues are ferricrocin, ferrioxamine B, N⁵-acetyl-N⁵-hydroxy-L-ornithine tripeptide derivatives and spermidine-catechol derivatives.

The invention furthermore relates to a process for the preparation of compounds of the formula I, which comprises linking a carrier residue, particularly preferably via a linker, to the aminomonosaccharide by a linkage reaction.

For the linkage, a carrier residue can be chemically modified with a linker, the aminomonosaccharide residue or a group composed of linker and aminomonosaccharide residue so that a functional group is present and a linkage reaction can take place. The introduction of a carboxyl group or its chemical equivalent is preferred for a modification of the carrier residue.

A chemical modification may mean a chemical activation of a functional group in the carrier residue (for example conversion of a carboxyl group into an acid chloride or anhydride or reaction with a carbodiimide, 2,6-dichlorobenzoyl chloride or 2,4,6-trichlorobenzoyl chloride) or attachment of a bifunctional compound. Examples of bifunctional compounds are aliphatic or aromatic dicarboxylic acids such as oxalic acid, malonic acid, succinic acid, maleic acid, fumaric acid, adipic acid, phthalic acid, aliphatic or aromatic amino carboxylic acids such as amino acids, aminopropionic acid, aminobutyric acid or nicotinic acid, hydroxy carboxylic acids such as hydroxyacetic acid, hydroxypropionic acid or hydroxybutyric acid, or a glycol such as mono-, oligo- or polyethylene glycol. It is possible to use for the chemical attachment of a bifunctional compound an appropriate activated compound as customary in organic synthesis. The bifunctional compound may also be a linker.

For example, succinic acid (as acid chloride or anhydride) can be linked to the terminal amino group in ferrioxamine B or the free hydroxyl group in ferricrocin in order to obtain a modified carrier residue.

It is possible to employ as linkage reaction for example an esterification, a transesterification, an amide formation, an ether formation, an alkylation, an N-alkylation, a Wittig reaction or a substitution.

A carrier residue which already has a suitable carboxyl group as coupling group or is correspondingly modified can be linked to the disaccharide component of a salmycin by transesterification, in which case the carrier residue of salmycin is replaced. For this purpose, salmycin A, B, C or D, which may be provided with protective groups, is reacted with an excess of the carrier residue.

The process of preparation may mean that the product contains protective groups. These protective groups can be removed by conventional methods, as also described in Th.W. Greene and P.G.M. Wuts, "Protective groups in organic synthesis", John Wiley & Sons, New York-Chichester-Brisbane-Toronto-Singapore 1991.

Compounds of the formula I may furthermore be prepared in analogy to the processes described by H. Zähner et al. (The Japanese Journal of Antibiotics, Vol. XXX Suppl. (1977) S-201) and A. Brochu et al. (Antimicrobial Agents and Chemotherapy 36 (1992) 2166-2175).

The carrier residues can be obtained from natural substances, especially antibiotics, or by synthesis.

A review of the synthesis of siderophores is given by W. Wierenga "The total synthesis of natural products", Vol. 4, Chapter 3, pages 274-286 and R.J. Bergeron, Chem. Rev. 84 (1984) 587-602.

The ferrichromes can be obtained from fungal cultures (details of this in: The Merck Index, 11th Edition, Merck & Co. Inc., N.J., USA, 1989, page 631). Ferricrocin is obtained as described by Kappner et al., Arch. Microbiol., 1977 (see also Fiedler and Sauerbier, Eur. J. Appl. Microbiol., 1977, both quoted by H. Zähner et al., The Japanese Journal of Antibiotics, Vol. XXX Suppl. (1977) S-201).

Ferrioxamines can be obtained from streptomyces cultures (see H. Bickel et al., Helv. Chim. Acta 43 (1960) 2119-2128). Synthesis of ferrioxamines B and D₁ is described by V. Prelog and A. Walser, Helv. Chim. Acta 45 (1962) 631.

The synthesis of N⁵-acetyl-N⁵-hydroxy-L-ornithine tripeptide and derivatives thereof is described by E.K. Dolence et al., J. Med. Chem. 34 (1991) 956-968 and 968-978; J. Med. Chem. 33 (1990) 461-464 (see also A. Brochu et al. (Antimicrobial Agents and Chemotherapy 36 (1992) 2166-2175).

Catechol-like iron(III) complexes can be obtained by means of Gram-negative bacteria (I.G. O'Brien, F. Gilson, Biochim. Biophys. Acta 215 (1970) 393). Syntheses of catechol-like siderophores are listed, for example, in W. Wierenga "The total synthesis of natural products", Vol. 4, Chapter 3, pages 274-286 and R.J. Bergeron, Chem. Rev. 84 (1984) 587-602. The latter publication describes the synthesis of acyclic enterobactin analogs. Catecholspermidine derivatives can be synthesized as described by McKee et al., Bioconjugate Chem. 2 (1991) 281-291 (quoted by A. Brochu et al. Antimicrobial Agents and Chemotherapy 36 (1992) 2166-2175).

Examples of suitable linkers are saccharides. Saccharides have the advantage that they can easily be cleaved off in the bacterial cell. Particularly suitable as linker are compounds of the formula II: 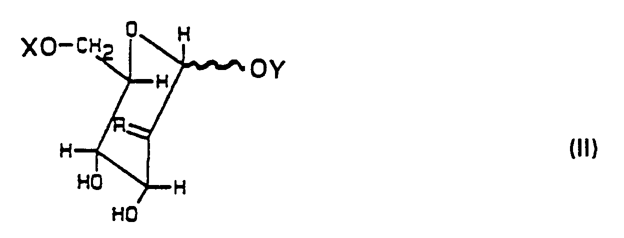
where R is O, (-OH)₂, OH and H, NH₂ and H, NOR' or NOH, where R' is phenyl or branched or unbranched alkyl with 1-10 carbon atoms, preferably with 1-6 carbon atoms, in particular with 1-3 carbon atoms, and X and Y are H.

The present invention likewise relates to the said compounds of the formula II, excepting the compound with R = O.

The compound of the formula II with R = O is known from the literature (see, for example, "Carbohydrates", Chapman and Hall). It is glucosone.

The present invention likewise relates to the preparation of the compounds of the formula II with R = NOH or (-OH)₂. The oxime can be obtained by hydrolysis of salmycins A or D or by synthesis. The oxime is synthesized preferably by reacting glucosone with hydroxylamine, for example at pH 4.5. The compound with R = (-OH)₂ corresponds to the acetal of glucosone. The acetal can be formed from glucosone in aqueous solution by conventional methods. Further compounds of the formula II can be obtained by hydrolysis of corresponding salmycin derivatives.

The invention furthermore relates to the aminomonosaccharide of the formula III, to a process for its preparation, and to the use as component of active substances, in particular of antibiotics. The aminomonosaccharide has the following structure: 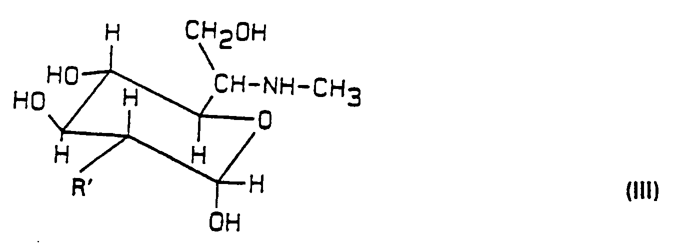
R' is -OH.

The process for preparing the compound of the formula III comprises a salmycin, or a salmycin derivative or a disaccharide derived therefrom being hydrolyzed or enzymatically cleaved after reduction.

The aminomonosaccharide is preferably obtained from salmycins A-D, for example by hydrolysis of the salmycins with aqueous trifluoroacetic acid at elevated temperature or reduction of the disaccharide residue of salmycins A-D or of the free disaccharide with subsequent enzymatic hydrolysis.

Reduction of the disaccharide may lead to either the glucose or mannose analog which can be hydrolyzed with a suitable glucosidase or mannosidase respectively.

The invention furthermore relates to disaccharides of the formula IV and to a process for their preparation. The disaccharides have the structure 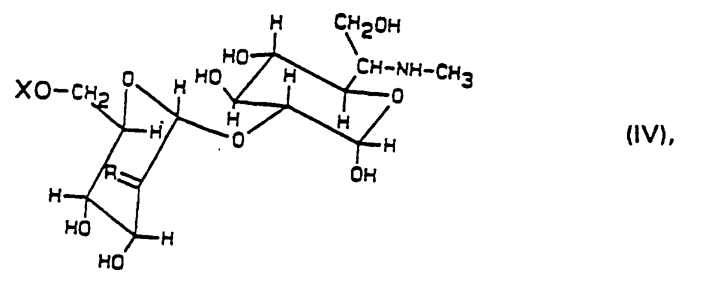
where R is O, (-OH)₂, OH and H, NH₂ and H, NOR' or NOH, where R' is phenyl or branched or unbranched alkyl with 1-10 carbon atoms, preferably with 1-6 carbon atoms, in particular with 1-3 carbon atoms, and X and Y are H.

Disaccharides of the formula IV are particularly preferred for the coupling to a carrier residue.

The process for the preparation of compounds of the formula IV comprises hydrolysis of a salmycin or of an appropriate salmycin derivative.

The disaccharides of the formula IV are preferably obtained by hydrolysis from salmycin A, B, C or D produced by fermentation. It is advantageous for this purpose, and with a view to a subsequent selective coupling to a carrier unit, to protect the hydroxyl groups of the salmycins used by protective groups such as benzyl, substituted benzyl, silyl or allyl radicals. The conditions for introducing such protective groups are known to the skilled worker and can be referred to in Th.W. Greene and P.G.M. Wuts, "Protective groups in organic synthesis", John Wiley & Sons, New York-Chichester-Brisbane-Toronto-Singapore 1991. The protected or free salmycin ought to be hydrolyzed under mild conditions, which can take place with dilute bases such as ammonium hydroxide solution at a pH of about 9.5 and at room temperature. Usually the hydrolysis has essentially taken place after two hours. The disaccharide can be isolated by chromatography such as reverse phase, adsorption or gel chromatography. If the initial salmycins were protected, the result is a disaccharide with only one free hydroxyl group, which can be used for a coupling.

Examples of aminosugar active substances are listed hereinafter. The residue R represents a disaccharide of the formula IV, and the carrier residue shown assumes the meaning of X.

Aminosugar active substance with ferrioxamine B as carrier residue: 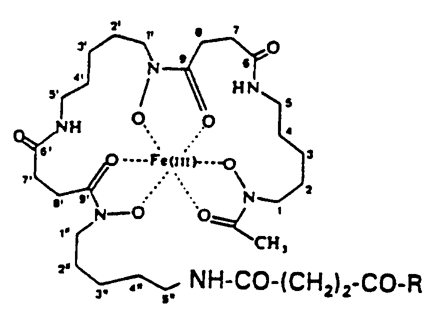
Aminosugar active substance with ferricrocin as carrier residue: 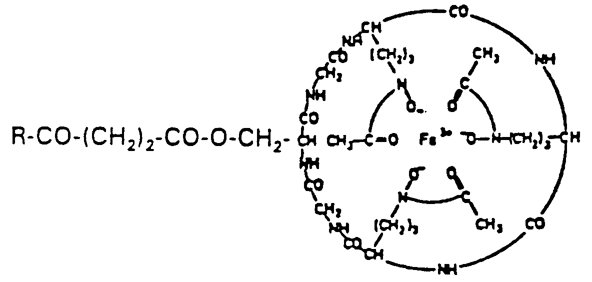
Aminosugar active substance with N⁵-acetyl-N⁵-hydroxy-L-ornithine tripeptide derivative as carrier residue (desferri form depicted): 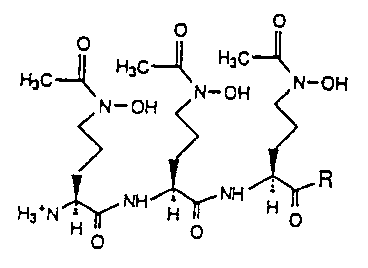
Aminosugar active substance with spermidine-catechol derivatives as carrier residue (desferri form depicted): 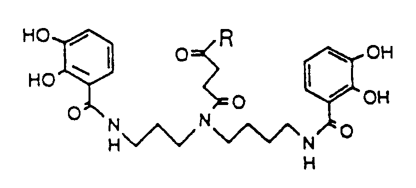
Derivatives of the salmycins can be prepared as follows.

### Hydroxy derivative:

The carbonyl group of salmycin B can be reduced with customary reducing agents or by cathodic reduction. The reaction is preferably carried out in aqueous solution at pH 7 with sodium borohydride (NaBH₄). The reduction yields two isomeric compounds. The products are separated and purified by chromatography, preferably by reversed phase chromatography with C₁₈-silica gel as stationary phase.

### Amino derivative:

The carbonyl group of salmycin B can be converted into an amino group by reductive amination. Preferably salmycin B is treated in the cold with a dry solution of ammonia in methanol (maximum 17% by weight of NH₃ in dry methanol at room temperature) and an equimolar amount of sodium borohydride (NaBH₄) with stirring. The amino product is formed within a few minutes. Solvent and excess ammonia can be evaporated off under reduced pressure. The product is purified by chromatography, preferably by reversed phase chromatography with C₁₈-silica gel as stationary phase. Two isomers are obtained.

### Oxime ether of salmycin B:

For the preparation of oxime ethers of salmycin B, phenoxy- or alkoxy-ammonium chloride, preferably methoxy-(CH₃ONH₃Cl) or ethoxy-ammonium chloride (C₂H₅ONH₃Cl), and an aqueous solution of salmycin B are mixed. The reaction is carried out preferably in a solution of pH 7 and at room temperature for several hours. The work-up of the solution comprises concentration and desalting. The product can be purified by chromatography, preferably by reversed phase chromatography with C₁₈-silica gel as stationary phase. The two possible oxime ethers are obtained.

The present invention also relates to a process for the preparation of the compounds of formula II by hydrolysis of the corresponding salmycin derivative. Hydrolysis may be performed preferably at pH 9.5 by adding a base such as 0.1M NaOH or KOH solution to an aqueous solution of the salmycin derivative at room temperature (approximately 2 h reaction time).

The invention furthermore relates to pharmaceuticals which contain one or more compounds of the formula I, their chemical equivalents or their physiologically tolerated salts and one or more vehicles or ancillary substances, such as, for example, fillers, emulsifiers, lubricants, flavorings, colorants or buffer substances, and which may be administered in a suitable pharmaceutical form such as, for example, tablets, capsules, a suspension or solution.

Examples of vehicles are tragacanth, lactose, talc, agar-agar, polyglycols, hydrocolloids, ethanol or water. Suitable and preferred for parenteral administration are, for example, suspensions or solutions in water. It is likewise possible to administer the active substance without additives in a suitable form such as capsules.

The following examples and the contents of the patent claims explain the present invention in more detail.

### Examples

### 1. Obtaining the salmycin

### Example 1.1: Cultivation of Streptomyces violaceus

Streptomyces violaceus (DSM 8286 deposited at the Deutsche Sammlung fur Mikrooganismen on May 13, 1993) is maintained on the following solid nutrient medium:

| | |
|---|---|
| Starch: | 10 g/l |
| Casein: | 1 g/l |
| Peptone: | 1 g/l |
| Yeast extract: | 1 g/l |
| Malt extract: | 10 g/l |
| K₂HPO₄: | 0.5 g/l |
| Agar: | 15 g/l |

The medium is sterilized at 121°C for 30 minutes, cooled to 45°C and dispensed into Petri dishes. The Petri dishes are inoculated with a spore suspension and incubated at 28°C for 8 days. Storage takes place at 4°C. 1 cm² of an incubated plate is used as inoculum for 500 ml of preculture of the following composition:

| | |
|---|---|
| Glucose: | 15 g/l |
| Soybean meal: | 15 g/l |
| Cornsteep liquor: | 5 ml/l |
| CaCO₃: | 2 g/l |
| NaCl: | 5 g/l |
| pH 7.2 | |

Incubation takes place shaking at a frequency of 240 rpm on a rotary shaker at 28°C for 2 days. 25 ml of the preculture are used as inoculum for a main culture (50 ml/ 300 ml Erlenmeyer flask) of the following composition:

| | |
|---|---|
| Soybean meal: | 20 g/l |
| Mannitol: | 20 g/l |
| pH 7.5 | |

The main culture is incubated at 28°C and 240 rpm for 3-4 days. A diffusion test after this time reveals a biological activity of a 21-23 mm diameter zone of inhibition of Staphylococcus aureus 209 P.

### Example 1.2: Production of salmycins A, B, C and D in a fermenter

Strain maintenance and preculturing of Streptomyces violaceus (DSM 8286) take place in analogy to Example 1.1. The main culture takes place in a 12 l fermenter with 9 l of main culture nutrient solution from Example 1.1. The inoculum comprises 1% of the fermenter volume. Incubation takes place at 28°C, 500 rpm and an aeration rate of 0.5 l/min for 2-3 days. The average biological activities achieved are 22-27 mm zones of inhibition of Staphylococcus aureus 209 P.

### Example 1.3: Isolation of crude salmycin

180 l of filtrate from cultures obtained in Example 1.2 are adjusted to pH 6.8 and loaded on to a column packed with 17 l of an adsorption resin. The adsorbent preferably used is a styrene/divinylbenzene copolymer in powder form, such as ®Diaion HP-20 (Mitsubishi Chem. Ind., Japan). The loaded support is then washed with deionized water. The salmycin complex is now eluted with a gradient containing 0-20% by volume isopropanol. Fractions are collected and tested for antibacterial activity. The salmycin-containing fractions are combined (about 30 l in total). To remove acidic impurities, the liquid is filtered through an anion exchanger column (for example 1 l of diethylaminoethyl-Sepharose such as DEAE-®Sephadex fast flow ion exchanger from Pharmacia Fine Chemicals AB, Sweden) which has previously been equilibrated with ammonium acetate solution, pH 7.0. The solution is subsequently concentrated by ultrafiltration using a semipermeable and perm-selective membrane such as, for example, a "®Nadir UF-CA-1" membrane (Hoechst; Frankfurt, Germany) and is freeze-dried. 38 g of salmycin crude product result.

### Example 1.4: Preliminary separation of the crude product mixture

35 g of the crude product mixture obtained in Example 1.3 are dissolved in 500 ml of distilled water and loaded onto a column of capacity 3.5 l (internal diameter 11.3 cm, height 36 cm) which is packed with an adsorbent based on styrene/divinylbenzene copolymer, such as, for example, "®MCI GEL CHP 20 P" (Mitsubishi Kasei Corp., Tokyo, Japan). It is washed with water and eluted with a water/10% by volume isopropanol in water gradient. Liter fractions are collected. Fractions containing salmycin B and C and fractions containing mainly salmycin A are obtained. Concentration in vacuo and freeze drying afford 3.2 g of crude mixture of salmycin B and C and 1.4 g of crude salmycin A.

### Example 1.5: Purification of salmycin B and C

The crude mixture of salmycin B and C obtained in Example 1.4 is passed through a column packed with molecular sieves (internal diameter 10 cm, height 52 cm). The column packing is composed, for example, of 4 l of ®Fractogel TSK HW-40 F (E. Merck, Darmstadt, Germany), a hydrophilic vinyl polymer for gel permeation chromatography for separating low molecular weight compounds such as dextrans in the molecular weight range 100-7000 g/mol.

A mixture of water and methanol (1:1) with the addition of 1% by volume acetic acid is used as eluent. Fractions containing salmycin B or C are combined and freeze-dried. 510 mg of a crude product which contains 82% by weight salmycin B and 3% by weight salmycin C are obtained.

### Example 1.6: Purification of salmycin A

1.4 g of crude salmycin A, which also contains salmycin D, are purified as described in Example 1.5 using a column packed with molecular sieves. The eluent comprises four parts of water and one part of ethanol. The fractions which contain salmycin A are combined. Concentration and freeze drying afford 240 mg of a crude product with 85% by weight salmycin A (salmycin D present).

### Example 1.7: Obtaining salmycin A and D pure

The salmycin A (240 mg) which was obtained in Example 1.6 and which also contains salmycin D is dissolved in distilled water and purified by reverse phase chromatography. A column with an internal diameter of 3.2 cm and a height of 25 cm is packed with 200 ml of C₁₈-silica gel such as, for example, ®Nucleosil 12 C₁₈AB (Macherey & Nagel, Düren, Germany). A water/acetonitrile gradient (0-10% by volume acetonitrile) is used as eluent. 25 ml fractions are collected. Fractions which contain salmycin A and salmycin D are combined in each case. Concentration and freeze drying afford 6 mg of salmycin D and 130 mg of product with 99% by weight salmycin A.

### Characterization of salmycin A:

a) High resolution FAB mass spectrometry:
   Molecular ion peek of M+H⁺: 1053.4050±0.0006 Dalton.
   Mass (calculated for M+H⁺, monoisotopic): 1053.4052 Dalton.
   Formula:

   C₄₁H₇₀FeN₇O₂₁
b) ¹H-NMR (desferri form): signal at 2.8 ppm in D₂O (N-methyl group), no other signals for methyl protons.
c) UV-vis of the aqueous solution (phosphate buffer, pH 7.0):
   end absorption and broad absorption around 430 nm (log ε = 3.3).

### Characterization of salmycin D:

a) ESI mass spectrometry (electron spray ionization):
   Molecular ion peak of M+H⁺: 1039.4 Dalton.
   Formula:

   C₄₀H₆₈FeN₇O₂₁
b) ¹H-NMR (desferri form): signal at 2.8 ppm in D₂O (N-methyl group), no other signals for methyl protons.
c) UV-vis of the aqueous solution (phosphate buffer, pH 7.0):
   end absorption and broad absorption around 430 nm (log ε = 3.3).

### Example 1.8: Obtaining salmycin B and C pure

The mixture of salmycin B and salmycin C obtained in Example 1.5 is purified by reverse phase chromatography as in Example 1.7. The eluent comprises a mixture of water, 6% by volume acetonitrile and 0.1% by volume trifluoroacetic acid. 402 mg of salmycin B and 8 mg of salmycin C are obtained.

### Characterization of salmycin B:

a) High resolution FAB mass spectrometry:
   Molecular ion peek of M+H⁺: 1038.3941±0.0007 Dalton
   Molecular ion peek of M+H⁺+H₂O: 1056.406±0.001 Dalton
   Mass calculated for M+H⁺H₂O: 1056.4053 Dalton
   Formula:

   C₄₁H₆₉FeN₆O₂₁
b) ¹H-NMR (desferri form): signal at 2.8 ppm in D₂O (N-methyl group), no other signals for methyl protons.
c) UV-vis of the aqueous solution (phosphate buffer, pH 7.0):
   end absorption and broad absorption around 427 nm (log ε = 3.3).

### Characterization of salmycin C:

a) ESI mass spectrometry (electron spray ionization):
   Molecular ion peak of M+H⁺: 1024.4 Dalton
   Molecular ion peak of M+H⁺+H₂O: 1042.4 Dalton
   Formula:

   C₄₀H₆₇FeN₆O₂₁
b) ¹H-NMR (desferri form): signal at 2.8 ppm in D₂O (N-methyl group), no other signals for methyl protons.
c) UV-vis of the aqueous solution (phosphate buffer, pH 7.0):
   end absorption and broad absorption around 430 nm (log ε = 3.3).

### Example 2: Preparation of disaccharides

100 mg of salmycin A, B, C or D or a corresponding amount of the form provided with protective groups are dissolved in 20 ml of water. The pH of the solution is adjusted to pH 9.5 by dropwise addition of 0.1 M NaOH solution. Hydrolysis takes place at room temperature and is substantially complete after 2 hours. The solution is then neutralized. The disaccharide or the protected compound is isolated by reversed phase chromatography (HPLC) on silica gel RP18 and with pure water as eluent.

### Example 3: Preparation of salmycin A-oxime methyl ether

100 mg of salmycin B are dissolved in 100 ml of phosphate buffer, pH 7, and 100 mg of O-methylhydroxyl-ammonium chloride (E. Merck, Art. No. 10 592) are added. Reaction time is 5 hours. Two forms of salmycin A-oxime methyl ether (syn and anti) are formed. They are isolated as described in Example 7. 41 mg of the syn form and 44 mg of the anti form are obtained.

The reaction is monitored with a HPLC system (®Nukleosil 7-C₁₈ AB; Macherey & Nagel, Düren, Germany) with a mixture of water/8.75 vol.-% acetonitrile/0.1 vol.-% trifluoroacetic acid as solvent.

### Example 4

### Reduction products of salmycin B

104 mg of salmycin B are dissolved in potassium phosphate buffer, pH 7.0, and 2 mg of sodium borohydride are added. The reaction mixture is left at room temperature. The course of the reaction is monitored by HPLC (column: ®Nukleosil 7-C₁₈ AB; (Macherey & Nagel); solvent: water/10 vol.-% acetonitrile/0.1 vol.-% trifluoroacetic acid). The reaction is almost complete after 2 hours. The reaction mixture is separated by preparative HPLC (column: 80 ml ®Nukleosil 10-C₁₈ AB (Macherey & Nagel); solvent: water/6 vol.-% acetonitrile/0.1 vol.-% trifluoroacetic acid).

Fractions are concentrated under reduced pressure and lyophilized to give 37 mg of mannosyl reduction product and 33 mg of glucosyl reduction product. These products appear as salts of trifluoroacetic acid with a purity of 97 and 95% by weight respectively.

Both compounds show molecular peaks M+H⁺ = 1040 Dalton in the FAB-MS spectra, which corresponds to the molecular formula

C₄₁H₇₁FeN₆O₂₁.

### Example 5

### Reductive amination of salmycin B

10 g of dry gaseous ammonia (NH₃)) are passed into 100 ml of dry methanol at -10°C. 100 mg of salmycin B dissolved in 10 ml of dry methanol, and 5 mg of sodium borohydride (NaBH₄) suspended in 5 ml of methanol are added to this solution. The reaction mixture is stirred for 10 minutes. Solvent and NH₃ are removed under reduced pressure. The residue is purified as described in Example 4. Two isomeric amino compounds are obtained, which are represented by formula Id.

## Claims

1. A compound of the formula I
X-B-Y (I)
where X is a carrier residue, B is a linker, which may also be omitted, and Y is an amino-monosaccharide residue, excepting salmycins A, B, C and D.

2. A process for preparing compounds of the formula I as claimed in claim 1, which comprises linking a carrier residue, particularly preferably via a linker to the amino monosaccharide by a linkage reaction.

3. A compound of the formula II 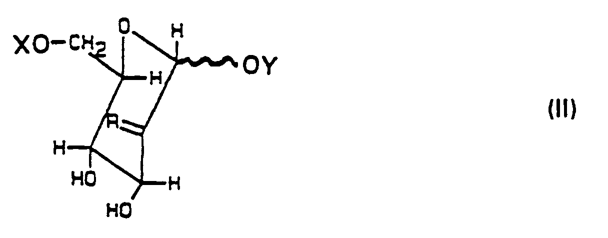 where R is (-OH)₂, OH and H, NH₂ and H, NOR' or NOH, where R' is phenyl or branched or unbranched alkyl with 1-10 carbon atoms, and X and Y are H.

4. A process for preparing a compound of the formula II with R = NOH as claimed in claim 3, which comprises hydrolyzing salmycin A or D or an appropriately substituted salmycin, or reacting glucosone with hydroxylamine.

5. A process for preparing a compound of the formula II with R = (-OH)₂ as claimed in claim 3, which comprises forming the acetal from glucosone.

6. A compound of the formula III 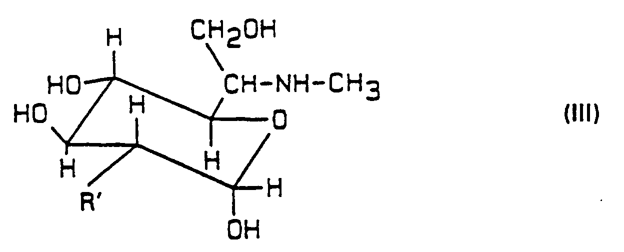 where R' is -OH.

7. A process for preparing compounds of the formula III as claimed in claim 6, which comprises a salmycin or a disaccharide derived therefrom being hydrolyzed or enzymatically cleaved after reduction.

8. A compound of the formula IV 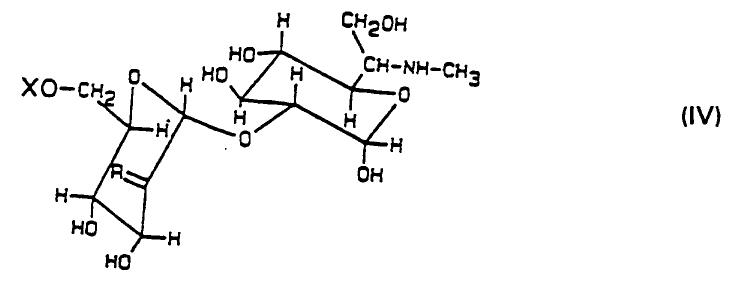 where R is (-OH)₂, OH and H, NH₂ and H, NOR' or NOH, where R' is phenyl or branched or unbranched alkyl with 1-10 carbon atoms, and X and Y are H.

9. A process for preparing compounds of the formula IV as claimed in claim 8, which comprises hydrolyzing a salmycin or an appropriately substituted salmycin.

10. A compound as claimed in claims 1, 3, 5 and 8 as pharmaceutical.

11. A pharmaceutical containing at least one compound as claimed in claims 1, 3, 5 and 8, where appropriate in addition to conventional ancillary substances and/or vehicles.

12. The use of compounds as claimed in claims 1, 3, 5 or 8 for preparing pharmaceuticals for the treatment of bacterial infections.

13. The use of compounds of the formula II as claimed in claim 3 as linker.
